# EUROPEAN PATENT SPECIFICATION

(11) **EP 4 149 251 B1**
(45) Date of publication and mention of the grant of the patent: **10.06.2026**
(21) Application number: 21726624.6
(22) Date of filing: 12.05.2021
(51) Int. Cl.: A01K 61/95, A01K 61/17, A01K 43/04, A01K 61/90

(54) **METHOD AND SYSTEM FOR IDENTIFYING FERTILIZED FISH EGGS**
VERFAHREN UND SYSTEM ZUR IDENTIFIZIERUNG BEFRUCHTETER FISCHEIER
PROCÉDÉ ET SYSTÈME POUR IDENTIFIER DES OEUFS DE POISSON FERTILISÉS

(30) Priority: 15.05.2020 NO 20200577; 06.10.2020 NO 20201084
(43) Date of publication of application: 22.03.2023
(73) Proprietor: Cryogenetics AS, 2317 Hamar (NO)
(72) Inventor: WOLLA, Steffen, 2407 Elverum (NO); GREVLE, Inger, 2316 Hamar (NO)
(74) Representative: Acapo Onsagers AS
(86) International application number: PCT/EP2021/062714
(87) International publication number: WO 2021/228993

(56) References cited:
- WO-A1-2019/001604
- WO-A1-2019/073943
- CN-U- 208 891 463
- US-A- 4 009 782

## Description

The present invention relates to a method of analysing fish eggs with the purpose of determining whether they are fertilized. The present invention also relates to an associated system.

The present invention also relates to an apparatus capable of analysing fish eggs with the purpose of determining whether they are fertilized.

In aquaculture fish farming, spermatozoa and eggs are mixed to allow the eggs to be impregnated and fertilized. In order to determine the success rate of the fertilization process, a sample of eggs may be drawn from the fertilized batch and analysed to determine the ratio between the number of fertilized eggs and the number of unfertilized ones, e.g. the fertilization rate.

CN208891463U discloses a fish egg removing device with which inactivated fish eggs in the hatching tank can be collected and identified in real time. The device comprises a hatching tank, an image collection device and a suction device, the image collection device and the suction device being located above the hatching tank. By using the image acquisition device, the proportion of inactivated fish eggs and can be acquired.

WO2019/073943A1 discloses a process for conveying, to a prescribed processing position, receiving grooves that receive roe one at a time. The receiving grooves are photographed from a direction intersecting a depth direction and a conveying direction, and an injection operation using a capillary is controlled by assessing whether or not to inject an unprocessed roe with a prescribed substance on the basis of predetermined conditions according to an injection assessment flow.

US4009782A discloses an apparatus for separating dead fish eggs from live fish eggs. The apparatus is designed to automatically interrogate the degree of opaqueness of each egg in a batch of fish eggs. A selector individually separates an individual fish egg from the batch and injects the separated egg into a fluid stream for delivery past an optical interrogator. Dead fish eggs which display a high degree of opaqueness are optically detected and are hydraulically separated from live eggs which show a low degree of opaqueness.

In teleost fish, a blastodisc located at an animal pole of the egg, close to an outer membrane of the egg, is formed shortly after fertilization, in which disc initial cell division, or cleavage, takes place. In some fish species, the outer membrane of the egg is opaque, thus making it difficult to detect early cleavage. Such species include species in the family Salmonidae, e.g. the Atlantic salmon (*Salmo salar*)*.*

One object of the present invention is to addresses this problem. Another object is to provide a useful method and an associated method of analysing fish eggs, especially with the purpose of determining whether the eggs are fertilized. A further object is to provide a beneficial apparatus capable of analysing fish eggs.

According to one aspect, the present invention provides a method as defined by claim 1 of identifying fertilized fish eggs in a sample of eggs taken from a batch of incubating fish eggs. The method comprises the steps of:
- within a period of time from initial fertilization of the batch, treating the sample with a fixative bringing an outer membrane of the eggs at least partly transparent and/or a blastodisc of the eggs at least partly opaque;
- capturing images of individual eggs of the sample depicting the blastodisc of the egg;
- analysing the images for detecting divided cells in the blastodisc; and
- identifying each egg associated with an image showing divided cells in the blastodisc as a fertilized egg.

The step of capturing images of individual eggs may comprise capturing a plurality of images of each egg from a plurality of directions, i.e. from different directions.

The step of capturing at least one image of individual eggs may comprise capturing a first set of images of each individual egg when rotated in a first plane and capturing a second set of images of the individual egg when rotated in a second plane. The second plane may be orthogonal to the first plane.

Capturing the first set of images may comprise capturing the first set of images with a first camera having an optical axis located in said first plane, and capturing the second set of images may comprise capturing the second set of images with a second camera having an optical axis located in said second plane.

Capturing any one of the first set of images and the second set of images may comprise capturing images of said individual egg when it is rotated an entire revolution. Advantageously, capturing any one of the first set of images and the second set of images may comprise rotating said individual egg between image captures within the range of 45° to 135°.

The step of capturing images of individual eggs may comprise capturing, in a single exposure, a plurality of images of each egg from a plurality of directions. One or a plurality of prisms and/or mirrors may be used to capture, in said single exposure, said images of the egg from said plurality of directions.

The step of capturing images of individual eggs may comprise bringing the eggs in single file passed an imaging system, i.e. guiding the eggs one-by-one through the imaging system.

The period of time from initial fertilization of the batch to treating the sample with the fixative may be within the range of 30 to 220 degree-hours. Degree-hours is a measure which takes into account the temperature in the incubator when timing fish egg development. In particular, degree-hours divided by the temperature in the incubator (in centigrades) will give a time measure. For example, if the temperature in the incubator is 10°C, the sample should preferably be treated with the fixative within the range of 30 degree-hours/10°C to 220 degree-hours/10°C = 3 to 22 hours post fertilisation. For most fish species this will give the eggs sufficient time to develop detectable cleavage in the blastodisc, e.g. 2 to 16 cells. When the sample is to be treated with the fixative may vary between species. For example, eggs of the species *S. salar* may advantageously be treated with the fixative within the range of 170 to 190 degree-hours, e.g. at 180 degree-hours.

The fixative may comprise acetic acid and may advantageously comprise any one of an acetic acid-alcohol-formaldehyde solution, such as Serra's fixative or Stockard's solution, or a non-formaldehyde acetic acid solution, such as the fixative marketed under the trade name AquaBoost^{®} Quattro.

The method may comprise the step of compressing the egg to a predetermined thickness prior to said step of capturing said at least one image of the egg. The predefined thickness may be any one of: within the range of 0.1 to 1.5 mm; within the range of 0.3 to 1.0 mm; within the range of 0.4 to 0.6 mm; and 0.5 mm. At least for eggs of the species *S. salar,* this may be an advantageous predefined thickness.

The step of compressing the egg may comprise the sub-steps of trapping the egg between two holding elements and bringing the holding elements together compressing the trapped egg. The holding elements may comprise ductile strips of transparent sheet material. The transparent sheet material may comprise regenerated cellulose, e.g. CELLOPHANE (TM). The step of capturing an image of the compressed egg may comprise the sub-steps off: arranging an image-capturing device on a first side of the compressed egg; arranging a light source on a second side of the compressed egg, said second side being opposite the first side; and transmitting light from the light source (427, 627) through the compressed egg and into the image-capturing device.

According to another aspect, the present invention provides a fish egg analysis systen as defined by claim 11 comprising:
- at least one imaging system being configured for capturing at least one image of individual fish eggs of a sample of eggs comprising fertilized and unfertilized fish eggs depicting the blastodisc of the eggs;
- an image analysis system being configured for analysing the captured images for detecting divided cells in the blastodisc and identifying each egg associated with an image showing divided cells in the blastodisc as a fertilized egg; and
- a conveyor system arranged for conveying the eggs of the sample past the at least one imaging system in single file.

The fish egg analysis system comprises a fixation system configured for mixing a sample of eggs comprising fertilized and unfertilized fish eggs with a fixative bringing an outer membrane of the eggs at least partly transparent and/or a blastodisc of the eggs at least partly opaque.

The at least one imaging system may comprise; a first camera being configured for capturing a first set of images of each of said individual fish eggs when rotated in a first plane; and a second camera being configured for capturing a second set of images of the individual fish egg when rotated in a second plane. The second plane may be orthogonal to the first plane. The first camera may have an optical axis located in said first plane and the second camera may have an optical axis located in said second plane.

The at least one imaging system may comprise first and second imaging systems being positioned one after the other along conveyor system.

The conveyor system may comprise a tubular conduit for conveying the eggs. The conduit may have an inner diameter D within the range of D_{egg} < D < 2·D_{egg}, where D_{egg} is the diameter of the eggs of the sample.

It has been found that such a system is suited for conveying eggs from species in the family Salmonidae. Eggs of the species *S. salar* normally have a diameter of 6-8.5 mm and the tubular conduit in a system for analysing *S. salar* eggs may have an inner diameter of approximately 10 mm. Eggs of the species *Salmo trutta* (brown trout) normally have a diameter of 4.2 to 5.8 mm and, consequently, the tubular conduit in a system for analysing *S. trutta* eggs may have an inner diameter of approximately 6 to 7 mm.

The at least one imaging system may be configured for capturing images of individual eggs from a plurality of directions and the imaging system or systems may comprise one or a plurality of mirrors or prism allowing a camera of the imaging system to capture, in a single exposure, a plurality of images of each egg from different directions.

The fish egg analysis system may comprise a pressing device arranged upstream of said at least one imaging system, said pressing device being configured to compress each egg to a predetermined thickness. The predetermined thickness may, at least for eggs of the species *S. salar,* be any one of: within the range of 0.1 to 1.5 mm; within the range of 0.3 to 1.0 mm; within the range of 0.4 to 0.6 mm; and 0.5 mm.

To determine whether an image depicts a blastodisc having divided cells, the image analysis system may employ an algorithm built by a machine learning system utilising mathematical models based on sample or training images of eggs known to be fertilized.

A method is provided of determining a fertilization rate of a batch of mixed fish spermatozoa and eggs, the method comprising the steps of:
- drawing a sample comprising a plurality of eggs from the batch;
- within a period of time from initial mixing of the spermatozoa and eggs, treating the sample with a fixative bringing an outer membrane of the eggs of the sample at least partly transparent and/or a blastodisc of the eggs at least partly opaque;
- capturing at least one image of individual eggs of the sample depicting the blastodisc of the egg;
- analysing the images with the purpose of detecting divided cells in the blastodisc;
- classifying each egg associated with an image showing divided cells in the blastodisc as a fertilized egg; and
- calculating the fertilization rate as a ratio between the number of eggs classified as being fertilized and the total number of eggs in the sample.

A fish egg analysis apparatus is provided comprising:
- an imaging system configured for capturing images of fish eggs;
- an image analysis system configured for analysing the images captured by the imaging system;
- a conveyor system arranged for guiding the eggs through the imaging system allowing the imaging system to capture at least one image of each individual egg; and
- a pressing device configured to compress each fish egg to a predetermined thickness prior to the conveyor system guiding the egg through the imaging system.

The apparatus may comprise a receptacle for receiving a sample of fish eggs, e.g. taken from a batch of fertilized fish eggs, and a conveyor system configured for transporting eggs from the receptacle to the imaging system. The conveyor system may comprise a rotatable disc having, along its periphery, a plurality of openings for receiving the eggs.

The conveyor system may further comprise a first holding element and a second holding element configured to guide the eggs through the imaging system. The holding elements may comprise a flexible and transparent strip of sheet material having a substantially rectangular shape. The sheet may be made from regenerated cellulose, e.g. CELLOPHANE (TM), but other flexible and transparent materials may be used. The conveyor system may comprise a first storage reel holding a storage of the first holding element and second storage reel holding a storage of the second holding element.

The conveyor system may comprise a transfer device which is configured to transfer eggs from the disc to the first holding element. The transfer device may comprise a picking element which is configured to pick the eggs from the openings and position them onto the first holding element between the first and second holding elements. The picking element may comprise a suction tube having a distal end which is configured, by means of a negative pressure in the suction tube, to pick an egg from the opening of the disc.

The pressing device may comprise a pressing head configured to press on the holding elements to compress and flatten eggs deposited therebetween by the transfer device.

Following drawings are appended to facilitate the understanding of the disclosure and the invention as it is defined in the claims:
Fig. 1 is a schematic illustration of a fish egg analysis system according to one embodiment of the invention;
Fig. 2 is a schematic illustration of a fertilized egg from a teleost fish species at an early development stage;
Fig. 3 is a flow chart schematically illustrating a method according to one embodiment of the invention;
Fig. 4 schematically illustrates an embodiment of an imaging system.
Fig. 5 is a schematic illustration of a fish egg analysis system according to a further embodiment of the invention;
Fig. 6 is a flow chart schematically illustrating a method according to one embodiment of the invention;
Fig. 7 illustrates a fish egg analysis apparatus according to an embodiment of the invention;
Fig. 8 illustrates the fish egg analysis apparatus according to Fig. 7 in an alternative perspective view; and
Fig. 9 shows a detailed part-view of the apparatus according to Fig. 7.
Fig. 10 illustrates a fish egg analysis apparatus according to a further embodiment of the invention;
Fig. 11 shows a detailed part-view of the apparatus according to Fig. 10 with covers removed for clarity;
Fig. 12 shows a detailed part-view of the apparatus according to Fig. 10 from an alternative angle;
Fig. 13 shows a detailed part-view of the apparatus according to Fig. 10 with a disc of a conveyor system of the apparatus removed for clarity;
Fig. 14 shows a further detailed part-view of the apparatus according to Fig. 10;
Fig. 15 is a flow chart schematically illustrating a method according to one embodiment of the invention.

In the drawings, like reference numerals have been used to indicate common parts, elements or features unless otherwise explicitly stated or implicitly understood by the context.

In the following, one or more specific embodiments of the invention will be described in more detail with reference to the drawings. However, it is specifically intended that the invention is not limited to the embodiments and illustrations contained herein but includes modified forms of the embodiments including portions of the embodiments and combinations of elements of different embodiments as come within the scope of the following claims. It should be appreciated that in the development of any such actual implementation, as in any engineering or design project, numerous implementation- specific decisions must be made to achieve the developer's specific goals, such as compliance with system and/or business-related constraints, which may vary from one implementation of the invention to another. Moreover, it should be appreciated that such a development effort might be complex and time consuming, but would nevertheless be a routine undertaking of design, fabrication and manufacture for the skilled person having the benefit of this disclosure.

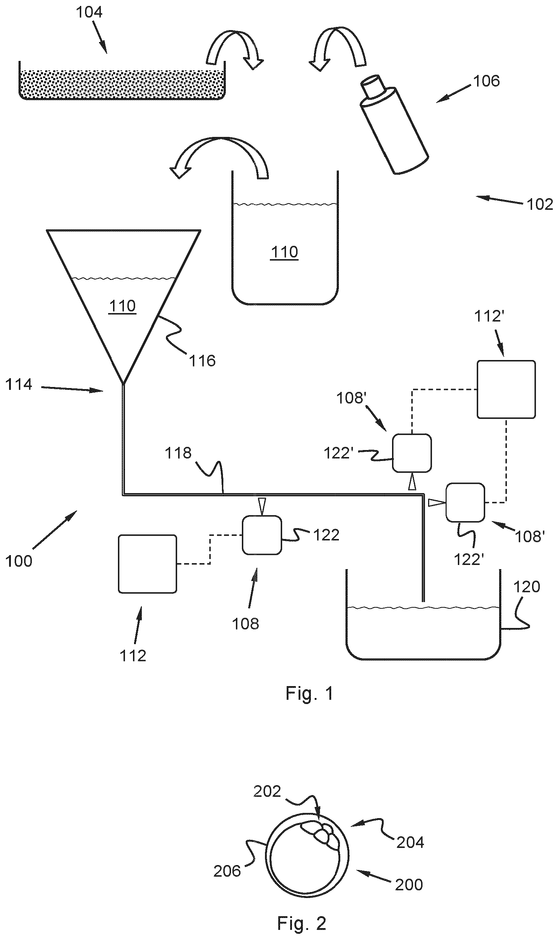

Fig. 1 illustrates, schematically, a fish egg analysis system 100 for detecting fertilized fish eggs and determining the fertilization rate of a batch of fertilized fish eggs. The system 100 comprise a fixation system 102 where a sample of eggs 104, drawn from the batch of mixed fish spermatozoa and eggs, is mixed with a fixative 106. The system 100 further comprises an imaging system 108 where images of the fixed eggs 110 are taken and an image analysis system 112 where the images are analysed. The system 100 also comprises a conveyor system 114 configured to pass the fixed eggs past the imaging system 108 so that one or a plurality of images can be taken of each egg.

The fixation system 102 may comprise a fixation station where the sample 104 and the fixative 106 are mixed manually. Alternatively, the fixation system 102 may comprise an automated fixations station where the fixative 106 is added to the sample 104 in an automated fashion.

In the shown embodiment, the conveyor system 114 comprises a funnel 116 configured for receiving the fixed eggs 110 and a tubular conduit 118 arranged at the bottom of the funnel 116. The conveyor system 114 also comprises a receptacle 120 into which the conduit 118 opens. Water is arranged to flow from the funnel 116, through the conduit 118 and to the receptacle 120, transporting the eggs through the system. A pump (not shown) may be used to pump water from the receptacle 120 back to the funnel 116. The eggs, however, are separated from the water and discarded once having arrived in the receptacle 120.

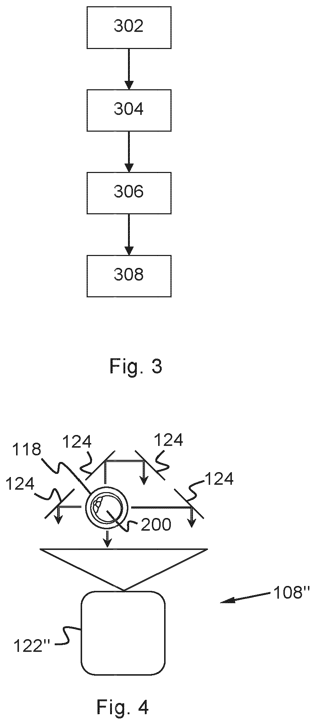

The tubular conduit 118 has an inner diameter D which is within the range of D_{egg} < D < 2·D_{egg}, where D_{egg} is the diameter of the eggs the system 100 is to analyse. In other words, the diameter of the conduit 118 is larger than the diameter of the eggs the system 100 is to analyse but smaller than two times this diameter, thus allowing the fixed eggs 110 to be brought through the conduit 118 in single file (also, see Fig. 4 which shows the conduit 118 in a cross-sectional view). For example, if the eggs to be analysed are Salmon eggs (*Salmo salar*), the inner diameter D of the conduit may be approximately 10 mm. It is understood that the system 100 may be configured in a modular fashion allowing for easy substitution of the conduit 118 to a conduit adopted to the species of eggs to be analysed.

The conduit 118 may be made from transparent plastic or glass.

The imaging system 108 is positioned adjacent the conduit 118 and comprises a camera 122 configured to take images of individual eggs as they pass. The camera 122 is connected to the image analysis system 112 allowing the images to be transferred to the same for analysis.

A method of determining a fertilization rate of a batch of mixed fish spermatozoa and eggs using the system 100 will now be discussed in more detail.

According to the method, fertilization is established by analysing and detecting cell division, or cleavage, in the eggs. In fish eggs, cleavage occurs only in a blastodisc, i.e. a thin region of yolk-free cytoplasm at an animal pole of the egg. Most of the rest of the egg cell is full of yolk. Fig. 2 schematically illustrates a fertilized egg 200 from a teleost fish species at an early development stage. The egg 200 comprises a blastodisc 202 having undergone initial cell cleavage to form a blastomere. In the illustration, the blastomere 202 has four cells. The blastomere 202 is positioned at an animal pole 204 of the egg, close to an outer membrane 206 of the egg.

The method comprises the initial step 302 (see Fig. 3) of drawing a sample comprising a plurality of eggs from the batch of mixed fish spermatozoa and eggs.

In a next step 304, the drawn sample is treated with a fixative bringing the outer membrane 206 of the eggs of the sample at least partly transparent and/or the blastodisc of the eggs at least partly opaque. Such fixatives are known in the art and may include acetic acid-alcohol-formaldehyde solutions, such as Serra's fixative. Other known fixtures are Stockard's solution and AquaBoost^{®} Quattro. However, any fixative capable of bringing the eggs transparent enough and/or the blastodisc visible enough so that cell development can be seen can be used.

The fixative will inhibit further cell development of the eggs and, consequently, the sample should not be treated with the fixative too early. Fertilized eggs must be allowed sufficient time to develop in order to enable them to be distinguished from un-fertilized eggs. The sample may be drawn from the batch and treated with the fixative at approximately 48 hours from initial mixing of the spermatozoa and eggs. During this period the blastodisc 202 of the fertilized eggs will typically have developed 2 to 16 cells forming a blastomere.

Generally, depending on the fish species and environmental factors, such as the temperature of the incubator, the sample may typically be drawn and treated with the fixative within a time period from initial mixing of the fish spermatozoa and the eggs within the range of 30 to 220 degree-hours. Eggs of the species *S. salar* may advantageously be treated with the fixative within the range of 170 to 190 degree-hours, e.g. at 180 degree-hours.

In a next step 306, the treated sample is entered into the conveyor system 114 (see Fig. 1) and the eggs are brought to pass the imaging system 108 in single file allowing the camera 122 to take one or a plurality of images of each egg. Since the blastodisc 202 is located at the animal pole 204 of the egg 200 (see Fig. 2), the system 100 may have, instead of a single imaging system 108, a plurality of imaging systems 108' positioned at different locations along the conduit 118, wherein each system 108' comprises a camera 122'. This will allow the eggs time to rotate between the systems 108', thus increasing the likelihood that at least one of the cameras 122' captures an image of the blastodisc 202. In such a configuration, it may be advantageous if the two imaging systems 108' are positioned on either side of a bend in the conduit 118, thereby further increasing the chances that the cameras 122' capture different sides of the egg.

In a further configuration, shown in Fig. 4, one or a plurality of mirrors or prisms 124 may be used to capture images of the egg from different directions using the same camera 122". In such an imaging system 108", a plurality of images of the egg from different directions can be captured simultaneously in a single exposure. In principle, such a configuration will allow the camera 122" to simultaneously capture images of the egg 200 from four orthogonal directions. If the configuration is employed in two imaging systems position on either side of a bend of the conduit 118, e.g. in the systems 108' in Fig. 1, it can almost be guaranteed that at least one of the captured images contains a clear view of the blastodisc 202 even if the egg, due to inertia, maintains its orientation in space throughout its travel through the conduit 118.

In a next step 308, the images of each egg are transferred to the image analysis system 112, 112' where the images are analysed for signs of normal cell division, i.e. cell division corresponding to normal development of the egg. If such cell division is detected in any of the images, the egg is classified as fertilized. If no cell division is detected, the egg is classified as un-fertilized.

The analysis may employ computer vision and algorithms built by machine learning systems utilising mathematical models based on sample or training images of eggs known to be fertilized.

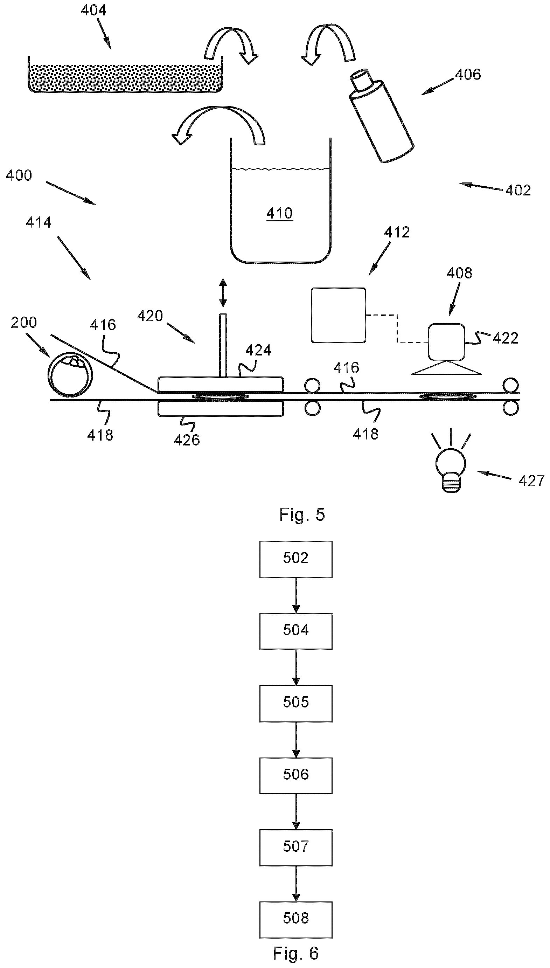

Fig. 5 schematically illustrates another embodiment of a fish egg analysis system 400 for detecting fertilized fish eggs and determining the fertilization rate of a batch of fertilized fish eggs.

The system 400 comprise a fixation system 402 where a sample of eggs 404, drawn from the batch of mixed fish spermatozoa and eggs, is mixed with a fixative 406. The fixation system 402 may be the same as described in Fig. 1, it may comprise a fixation station where the sample 404 and the fixative 406 are mixed manually or, alternatively, the fixation system 402 may comprise an automated fixations station where the fixative 406 is added to the sample 404 in an automated fashion.

The system 400 further comprises an imaging system 408 where images of the fixed eggs 410 are taken and an image analysis system 412 where the images are analysed.

The system 400 also comprises a conveyor system 414 configured to pass the fixed eggs past the imaging system 408 so that one or a plurality of images can be taken of each egg. In the shown embodiment, the conveyor system 414 comprises a first holding element 416 and a second holding element 418 configured to trap the fixed eggs between them. Each holding element 416, 418 may comprise a longitudinal strip of a flexible sheet material and at least one of the holding elements 416 may be made from a transparent material. The holding elements 416, 418 may for example be made from sheets of regenerated cellulose, e.g. CELLOPHANE (TM).

The system 400 also comprises a pressing device 420 configured to press onto the holding elements 416, 418 to compress and flatten eggs trapped therebetween. The pressing device 420 may comprise a pressing head 424 arranged on one side of the holding elements acting against support element 428 arranged on the other side of the holding elements. The pressing device 420 operates on the holding elements 416, 418 and an egg trapped therebetween to compress the egg. The compressive action should preferably be sufficiently large to allow the outer membrane of the egg to rupture but sufficiently small not to damage the blastomere, i.e. the blastodisc under cleavage. This will allow cytoplasm to escape the outer cell membrane but leave the blastomere inside the flattened egg.

It is understood that the thickness to which the egg should preferably be compressed may vary between egg species. For eggs of the species *S. salar,* the predefined thickness may preferably be within the range of 0.1 to 1.5 mm; more preferably within the range of 0.3 to 1.0 mm; and even more preferably within the range of 0.4 to 0.6 mm, e.g. 0.5 mm.

In the pressing device 420, the holding elements 416, 418 becomes orientated parallel to each other with the flattened egg housing the blastomere trapped between them.

After the pressing device 420, the flattened egg is transported between the holding elements 416, 418 to the imaging system 408. The imaging system 408 is positioned adjacent the holding elements 416, 418 and comprises a camera 422 oriented with its focal plane trained between the parallel holding elements 416 and 418, i.e. coinciding with the flattened egg. The camera 422 is configured to take at least one image of each flattened egg as the eggs are guided through the imaging system 408, thus also capturing images of the blastodiscs/blastomeres of the eggs.

The imaging system 408 may comprise an illumination device 427 arranged on a side of the holding elements 416, 418 being opposite the camera 422. In such a configuration, it is advantageous that both holding elements 416 and 418 are made from a transparent material, thus allowing transmission light microscopy techniques to be used for capturing the image. However, other illumination techniques may be used.

The camera 422 is connected to the image analysis system 412 allowing the images to be transferred to the same for analysis.

A method of determining a fertilization rate of a batch of mixed fish spermatozoa and eggs using the system 400 will now be discussed in more detail with reference to Fig. 6.

The method comprises the initial step 502 of drawing a sample comprising a plurality of eggs from the batch of mixed fish spermatozoa and eggs. In a next step 504, the drawn sample is treated with a fixative bringing the outer membrane 206 of the eggs (see Fig. 2) of the sample at least partly transparent and/or the blastodisc of the eggs at least partly opaque. As previously discussed, such fixatives are known in the art and may include acetic acid-alcohol-formaldehyde solutions, such as Serra's fixative. Other known fixtures are Stockard's solution and AquaBoost^{®} Quattro. However, as previously stated, any fixative capable of bringing the eggs transparent enough and/or the blastodisc/blastomere visible enough so that cell development can be seen can be used.

As also previously discussed, the fixative will inhibit further cell development of the eggs and, consequently, the sample should not be treated with the fixative too early. Fertilized eggs must be allowed sufficient time to develop in order to enable them to be distinguished from un-fertilized eggs. The sample may be drawn from the batch and treated with the fixative at approximately 48 hours from initial mixing of the spermatozoa and eggs. During this period the blastodisc 202 of the fertilized eggs will typically have developed 2 to 16 cells. As in the method previously discussed with reference to Fig. 3, the sample may typically be drawn and treated with the fixative within a time period from initial mixing of the fish spermatozoa and the eggs within the range of 30 to 220 degree-hours. Eggs of the species *S. salar* may advantageously be treated with the fixative within the range of 170 to 190 degree-hours, e.g. at 180 degree-hours. Eggs of other species may, depending on the fish species and environmental factors, such as the temperature of the incubator, be treated with the fixative within other ranges.

In a next step 505, the treated sample is entered into the conveyor system 414 (see Fig. 5) and in a subsequent step 506, the eggs are compressed and flattened in the pressing device 420 (see Fig. 5), thereby allowing cytoplasm to escape the outer cell membrane but leaving the blastomere inside the flattened egg, as previously discussed.

The fixative may affect the viscosity of cytoplasm (make it more viscous). Therefore, in order to press cytoplasm out of the egg in a controlled manner, it may be advantageous to effectuate the pressing step within a predefined time period from said step of treating the drawn sample with the fixative. For eggs of the species *S. salar,* it may be advantageously to effectuate the pressing step no later than 2 to 3 hours from treating the drawn sample with the fixative.

In a next step 507, the eggs are brought to pass the imaging system 408 in single file allowing the camera 422 to take one or a plurality of images of each egg. Since the blastodisc 202 is orientated substantially in a plane parallel to the holding elements 416, 418 (as a consequence of the egg having been compressed and flattened in the pressing device 420), the blastodisc/blastomere 202 will be positioned in the focal plane of the camera, thus allowing a clear image of the blastodisc/blastomere 202 to be captured.

In a next step 508, the images of each egg are transferred to the image analysis system 412 where the images are analysed for signs of normal cell division, i.e. cell division corresponding to normal development of the egg. If such cell division is detected in an image, the egg associated with that image is classified as fertilized. If no cell division is detected in the image, the egg is classified as un-fertilized.

The analysis may employ computer vision and algorithms built by machine learning systems utilising mathematical models based on sample or training images of eggs known to be fertilized.

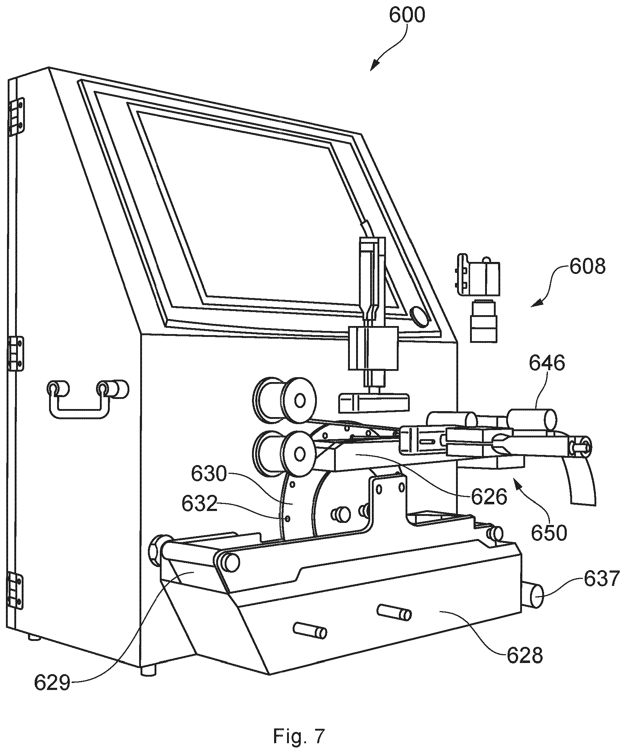

Figs. 7-9 show an embodiment of a fish egg analysis apparatus 600 which may be used in the above-discussed system for determining the fertilization rate of a batch of fertilized fish eggs.

The apparatus 600 comprises a receptacle 628 including an inlet 629 for receiving a sample of fish eggs, e.g. taken from a batch of fertilized fish eggs. In the present embodiment, the receptacle 628 has the form of a substantially rectangular open-top tray.

The apparatus 600 also comprise an imaging system 608 including an image-capturing device in the form of a camera 622 and an image analysis system. The image analysis system is housed inside the apparatus 600 and, therefore, is not visible in the figures.

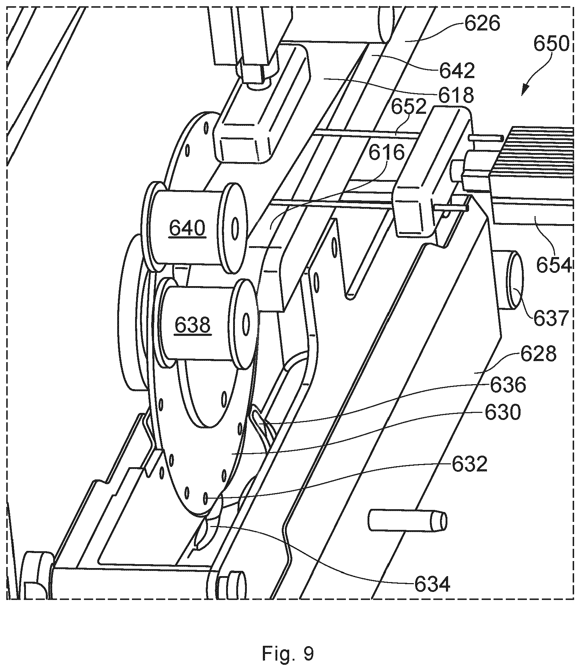

The apparatus 600 further comprises a conveyor system 614 configured to transport eggs from the receptacle 628 to the imaging system 608. The conveyor system 614 comprises a rotatable disc 630 having, along its periphery, a plurality of openings 632. The disc 630 is rotatably arranged about a substantially horizontal axis extending above and across the receptacle 628. The disc 630 has a diameter which is sufficient to allow the disc 630 to extend down into the receptacle 608 and allow eggs fed to the receptacle to be captured by the openings 632 when they are in a lower-most position during rotation of the disc (see Fig. 9).

The receptacle 628 may comprise one or a plurality of guide groves 634 configured for guiding the eggs towards and into the openings and a spillway passage 636 allowing fluid in which the eggs are transported into the receptacle 628 to be separated from the eggs and conducted to an outlet 637 and ejected. The disc 630 may be recessed in a side-wall of the receptacle 628, e.g. as is shown in Fig. 9.

For eggs of the species *S. salar,* experiments have shown that the openings 632 may have a diameter of approximately 6.5 mm and the disc may have a thickness of approximately 3 mm to allow effective capture of the eggs. Generally, however, it is understood that the dimensions of the openings 632 should be adapted to the species of eggs to be analysed.

The conveyor system 614 further comprises a first holding element 616 and a second holding element 618 configured to guide the eggs through the imaging system 608. In the present embodiment, both holding elements 616, 618 comprise a flexible and transparent strip of sheet material having a substantially rectangular shape. The sheet may be made from regenerated cellulose, e.g. CELLOPHANE (TM), but other flexible and transparent materials may be used. The conveyor system 614 comprises a first storage reel 638 holding a storage of the first holding element 616 and second storage reel 640 holding a storage of the second holding element 618.

The conveyor system 614 comprises a substantially rectangular support element 626 extending from above the receptacle 628 and through the imaging system 608. The support element 626 displays a substantially horizontal, upward-facing support surface 642 on which the first holding element 616, running from the first storage reel 638, and eventually also the second holding element 618, running from the second storage reel 640, are supported. The support element 626 extends substantially parallel to the disc 630 with the support surface 642 extending substantially orthogonal to the rotational plane of the disc at a level between the apex and the centre of the disc.

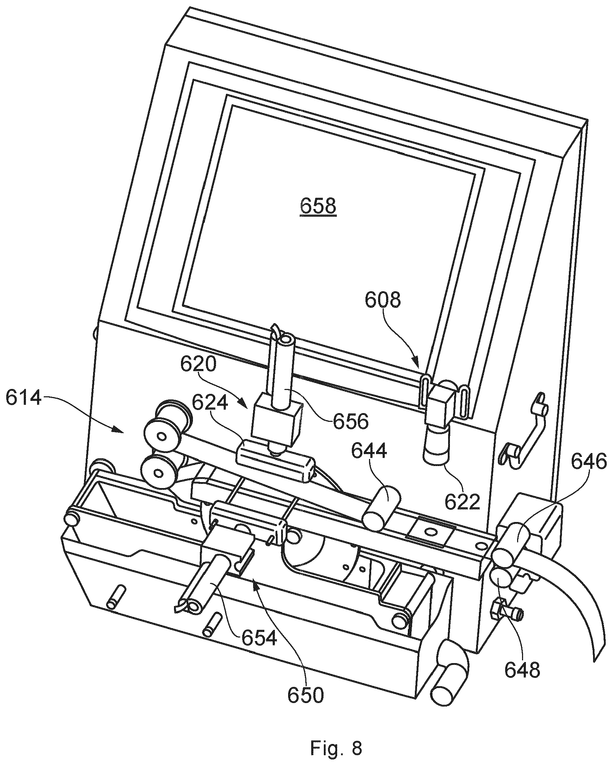

A guide roll 644 (see Fig. 8) forms a nip with the support surface 642 through which the holding elements 616, 618 are guided. A pair of drive rolls 646, 648, arranged at a downstream end of the support element 626, are arranged to advance the holding elements 616, 618 through the imaging system 608.

The conveyor system 614 further comprises a transfer device 650 which is configured to transfer eggs, raised by the disc 630, from the disc 630 to the first holding element 616 (see Figs. 7 and 9). The transfer device 650 comprises a picking element 652 (see Fig. 9) which is configured to pick the eggs from the openings 632 and position them onto the first holding element 616 upstream of the guide roll 644 and between the first and second holding elements 616, 618, as can be seen most clearly in Fig. 7. In the present embodiment, the picking element comprises a pair of suction tubes which are movable in the axial direction of the disc 630 by means of a pneumatically operated cylinder-piston arrangement 654.

The system 600 also comprises a pressing device 620 configured to compress and flatten the eggs prior to them entering the imaging system 608 (see Fig. 8). The pressing device 620 is arranged between the storage reels 638, 640 and the guide roll 644 and comprises a pressing head 624 arranged above the first holding element 616. The pressing head 624 is movable in a vertical direction by means of a pneumatically operated cylinder-piston arrangement 656 and is arranged to interact with the support device 626 at the position of the transfer device 650 to press onto the holding elements 616, 618 to compress and flatten the eggs deposited therebetween by the transfer device 650.

In operation, a suspension comprising eggs to be analysed is entered into the receptacle 628 through the inlet 629. In the receptacle 628, eggs are captured by the openings 632 in the rotating disc 630 when they are in a lower-most position. The fluid in which the eggs are transported into the receptacle 628 is conducted to the outlet 637 and ejected (see Fig. 9).

The rotating disc 630 raises the captured eggs above the support element 626 and the suction tubes 652 of the transfer device 650 are extended towards the disc 630 by the cylinder-piston arrangement 654. A distal end of each suction tube 652 is brought into contact with an egg positioned in an opening (see also Fig. 7). A negative pressure in the suction tube 652 attaches the egg to the distal end and the suction tube is then retracted to position the distal end above the first holding element 616. The negative pressure in the suction tube is then released and the egg deposited onto the first holding element 616. As can most clearly be seen in Fig. 7, the transfer device 650 positions the eggs onto the first holding element 616 upstream of the guide roll 646 and between the first and second holding devices 616, 618.

When the transfer device 650 has positioned the eggs onto the first holding element 616, the pressing head 624 of the pressing device 620 is lowered and the egg or eggs trapped between the holding devices 616, 618 are compressed and flattened to a predefined thickness. As previously discussed, for eggs of the species *S. salar,* the predefined thickness may preferably be within the range of 0.1 to 1.5 mm; more preferably within the range of 0.3 to 1.0 mm; and even more preferably within the range of 0.4 to 0.6 mm, e.g. 0.5 mm.

The first and second holding devices 616, 618 may be heat-sealed to each other during the pressing operation, joining the holding devices 616 and 618 to each other along a path that continuously or discontinuously encircles the flattened egg.

When the pressing device 620 has compressed and flattened the eggs (and possibly also heat-sealed the holding devices 616, 618), the drive rolls 646, 648 advance the holding devices 616, 618 and the flattened egg trapped therebetween to the guide roll 644 and, subsequently, to the imaging system 608. The guide roll 644 may preferably provide a nip with the support surface 642 that corresponds to the pressing thickness of the pressing device 620. In the imaging system, the camera 622 takes one or a plurality of images of each flattened egg. The imaging system 608 may comprise an illumination device 627 arranged below the first holding device 616, allowing light to be transmitted from the illumination device 627 through the flattened egg and into the camera 622. The illumination device 627 may advantageously be recessed in the support surface 624.

The images captured by the camera 622 are transferred to the image analysis system to be analysed. The image analysis system may comprise a central processing unit (CPU), volatile and/or non-volatile memory and data input and output devices. In the disclosed embodiment, the apparatus comprises an output device 658 in the form of a computer screen.

If the apparatus is used to determine the fertilization rate of the analysed eggs, the image analysis system analyses the images for signs of normal cell division, i.e. cell division corresponding to normal development of the egg. If such cell division is detected in an image, the egg associated with that image is classified as fertilized. If no cell division is detected in the image, the egg is classified as un-fertilized. As previously described, this analysis may employ computer vision and algorithms built by machine learning systems utilising mathematical models based on sample or training images of eggs known to be fertilized.

The eggs entering the receptacle 628 may be fixed eggs, i.e. eggs that have been subjected to a fixation operation as previously disclosed. However, for species of eggs where the blastodisc is visible without having been fixed, such an operation may be dispensed with.

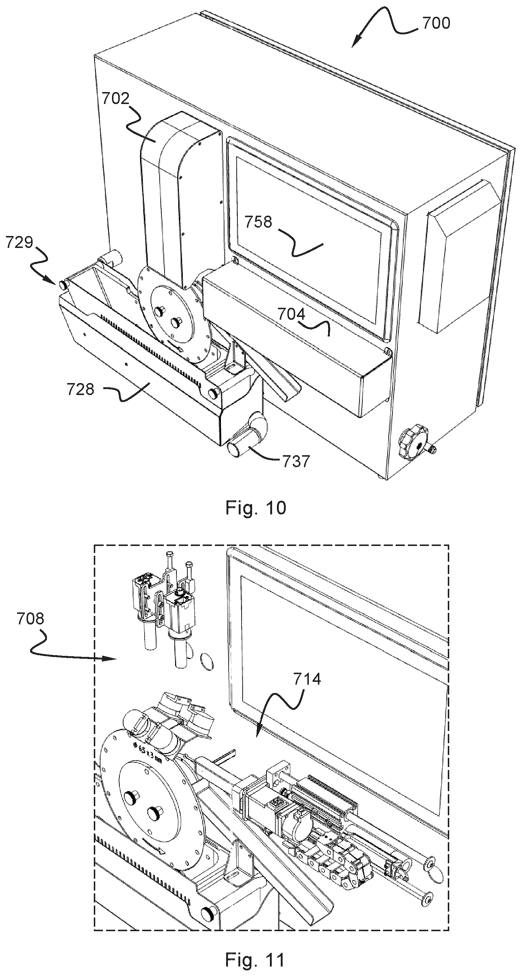

Figs. 10-14 illustrate yet another embodiment of a fish egg analysis apparatus 700 which may be used in a system for determining the fertilization rate of a batch of fertilized fish eggs. Fig. 10 shows the apparatus 700 in an isometric view and Fig. 11 shows a detailed part-view of the apparatus 700 with cover panels 702 and 704 removed for clarity.

Similar to the apparatus 600, the apparatus 700 comprises a receptacle 728 including an inlet 729 for receiving a sample of fish eggs, e.g. taken from a batch of fertilized fish eggs. In the present embodiment, the receptacle 728 has the form of a substantially rectangular open-top tray, similar to the receptacle 628.

The apparatus 700 also comprise an imaging system 708 (see Fig. 11), including an image-capturing device and an image analysis system, and a conveyor system 714 configured for transporting the eggs from the receptacle 728 to the imaging system 708.

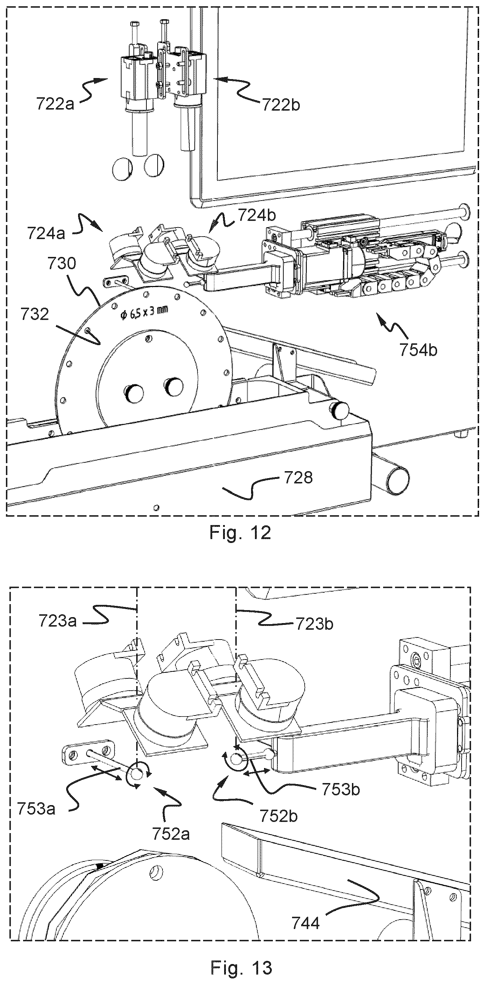

With reference to Fig. 12, which shows a detailed part-view of the apparatus 700 from a second viewing angle, the image-capturing device comprises a first camera 722a arranged in a first imaging station and an associated first lighting system 724a and a second camera 722b arranged in a second imaging station and an associated second lighting system 724b. The image analysis system is housed inside the apparatus 700 in the present embodiment and, therefore, is not visible in the figures.

Similar to the previously discussed conveyor system 614, the conveyor system 714 comprises a rotatable disc 730 (see Fig. 12) having, along its periphery, a plurality of through-openings 732. The disc 730 is rotatably arranged about a substantially horizontal axis extending above and across the receptacle 728. The disc 730 has a diameter which is sufficient to allow the disc 730 to extend down into the receptacle 728 and allow eggs fed to the receptacle to be captured by the openings 732 when the openings are in a lower-most position during rotation of the disc.

Similar to the receptacle 628 (see Fig. 9), the receptacle 728 may comprise one or a plurality of guide groves configured for guiding the eggs towards and into the openings and a spillway passage allowing fluid in which the eggs are transported into the receptacle to be separated from the eggs and conducted to an outlet and ejected. Similar to the disc 630, the disc 730 may be recessed in a side-wall of the receptacle 728.

For eggs of the species *S. salar,* experiments have shown that the openings 732 may have a diameter of approximately 6.5 mm and the disc may have a thickness of approximately 3 mm to allow effective capture of the eggs. Generally, however, it is understood that the dimensions of the openings 732 should be adapted to the species of eggs to be analysed.

With reference to Fig. 13, which shows a detailed part-view of the apparatus 700 with the disc 730 removed for clarity, the conveyor system 714 further comprises a first picking element 752a which is configured to pick an egg from an opening 732 in the disc 730 and position the egg in front of the first camera 722a in the first imaging station. In the present embodiment, the first picking element 752a comprises a substantially rectilinear first suction tube or straw 753a which extend substantially orthogonal to the optical axis 723a of the first camera 722a and also, in this embodiment, substantially parallel to the rotational axis of the disc 730, i.e. substantially orthogonal to the disc 730.

The conveyor system 714 further comprises a second picking element 752b which is configured to pick an egg from the first picking element 752b and position the egg in front of the second camera 722b in the second imaging station. In the present embodiment, the second picking element 752b comprises a substantially rectilinear second suction tube or straw 753b which extends substantially orthogonal to the optical axis 723b of the second camera 722b and also substantially orthogonal to the first suction tube 752a, i.e. substantially parallel to the disc 730 in this embodiment.

The first picking element 752a is operable by means of a first motor and pneumatic arrangement. In the present embodiment, the first motor and pneumatic arrangement is housed inside the apparatus 700 and, therefore, is not visible in the figures. The second picking element 752b is actuatable by means of a second motor and pneumatic arrangement 754b (see e.g. Fig. 12).

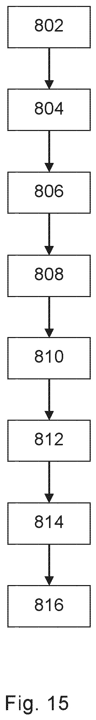

By means of the first motor and pneumatic arrangement, the first suction tube 753a is movable in its axial direction and preferably also rotatable about its axis, as is illustrated in Figs. 13 and 15. Likewise, by means of the second motor and pneumatic arrangement 754b, the second suction tube 753b is movable in its axial direction and preferably also rotatable about its axis, as is also illustrated in Fig. 13 and 16.

With further reference to Fig. 17, an embodiment of operating the apparatus 700 and the system in which it is arranged will now be discussed in more detail.

Operation of the apparatus 700 may involve an initial step 802 of drawing a sample comprising a plurality of eggs from the batch of mixed fish spermatozoa and eggs.

In a next step 804, the dsrawn sample may be treated with a fixative bringing the outer membrane 206 of the eggs (see Fig. 2) of the sample at least partly transparent and/or the blastodisc of the eggs at least partly opaque. As previously discussed, such fixatives are known in the art and may include acetic acid-alcohol-formaldehyde solutions, such as Serra's fixative. Other known fixtures are Stockard's solution and AquaBoost^{®} Quattro. However, as previously stated, any fixative capable of bringing the eggs transparent enough and/or the blastodisc/blastomere visible enough so that cell development can be seen can be used.

As also previously discussed, the fixative will inhibit further cell development of the eggs. Therefore, it may be advantageous to allowed the fertilized eggs sufficient time to develop prior to treating them with the fixative in order to enable fertilized eggs to be distinguished from un-fertilized.

In a next step 806, the treated sample is entered into the receptacle 728 through the inlet 729 (see Fig. 10).

In a next step 808, the eggs are entered into the conveyor system 714 (se Fig. 11) to be brought through the imaging system 708. In this step the eggs are captured by the openings 732 in the rotating disc 730 when the openings are in a lower-most position. The fluid in which the eggs are transported into the receptacle 728 is conducted to an outlet 737 (see Fig. 10) and ejected.

The rotating disc 730 raises the captured eggs to the position of the first picking element 752a and the first suction tube 753a is extended towards the disc 730 by the first motor and pneumatic arrangement (not visible). A distal end of the suction tube 753a is brought into contact with an egg positioned in an opening 732 and a negative pressure in the suction tube 753a attaches the egg to the distal end. The suction tube 753a is then retracted to position the distal end and the captured egg 200 in front of the first camera 722a such that the egg 200 becomes positioned on the optical axis 723a of the first camera 722a.

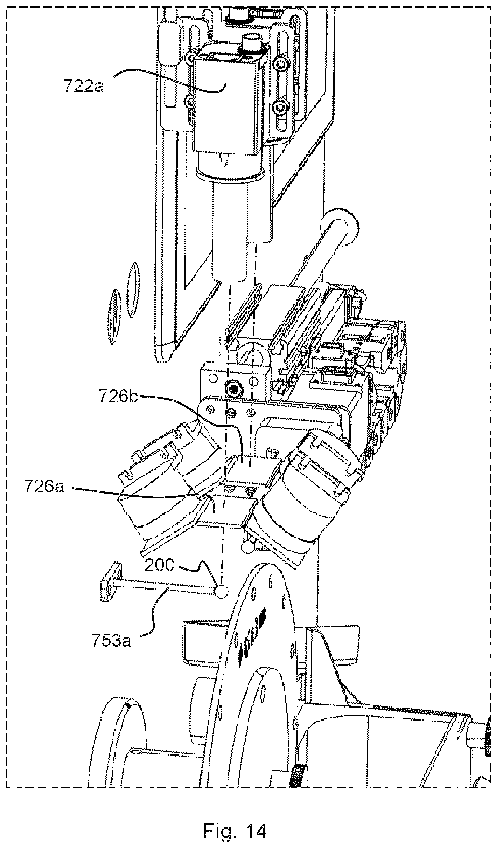

In a next step 810, the first camera 722a captures a first set of images of the egg. In the present embodiment, the images are captured through a protective glass sheet 726a (see Fig. 14). Between capture of each image in the first set of images, the suction tube 753a is rotated a predetermined angle about its axis, thus allowing images of the egg 200 to be captured from a plurality of directions in a plane which is orthogonal to axis of rotation of the first suction tube 753a. Advantageously, the first set of images include images of the egg 200 when rotated an entire revolution, i.e. throughout 360°. For example, the first set of images may comprise four images taken from four orthogonal directions, in which case the suction tube 753a and the attached egg 200 is rotated 90° between each image capture. However, the first set of images may involve a different configuration, e.g. one image captured every 180° (totalling 2 images in the set), one image captured every 120° (totalling 3 images in the set), one image captured every 72° (totalling 5 images in the set), etc.

The images of the first set of images are forwarded to the image analysis system, either individually one by one as they are captured or as a complete set in a single transfer after all the images of the set have been captured.

In a next step 812, the egg 200 is transferred from the first to the second imaging station. This is accomplished by the second motor and pneumatic arrangement 754b extending the second suction tube 753b towards the first second suction tube 753a until a distal end of the second suction tube 753b is brought into contact with the egg 200 which, at this point in time, is still attached to the distal end of the first suction tube 753a. A negative pressure is then established in the second suction tube 753b attaching the egg 200 to the distal end of the second suction tube 753b. The negative pressure in the first suction tube 753a is then released and the egg 200 is transferred to the second suction tube 753b. The second suction tube 753b is then retracted to position the distal end of the second suction tube 753b and the captured egg 200 in front of the second camera 722b such that the egg 200 becomes positioned on the optical axis 723b of the second camera 722b.

In a next step 814, the second camera 722b captures a second set of images of the egg 200. In the present embodiment, the images are captured through a protective glass sheet 726b (see Fig. 14). Between capture of each image in the second set of images, the suction tube 753b is rotated a predetermined angle about its axis, thus allowing images of the egg 200 to be captured from a plurality of directions in a plane which is orthogonal to the axis of rotation of the second suction tube 753b. Since the second suction tube 753b extends substantially orthogonally to the first suction tube 752a, the plane in which the second set of images are captured is substantially orthogonal to the plane in which the first camera 722a captured the first set of images.

As the first set of images, the second set of images may advantageously include images of the egg 200 when rotated an entire revolution, i.e. throughout 360°. For example, as the first set of images, the second set of images may comprise four images taken from four orthogonal directions, in which case the suction tube 753b and the attached egg 200 is rotated 90° between each image capture. Again, however, the second set of images may involve a different configuration, e.g. one image captured every 180° (totalling 2 images in the set), one image captured every 120° (totalling 3 images in the set), one image captured every 72° (totalling 5 images in the set), etc.

The images of the second set of images are forwarded to the image analysis system, either individually one by one as they are captured or as a complete set in a single transfer after all the images of the set have been captured.

Once the second set of images has been captured by the second camera 722b, the second suction tube 753b may be further retracted and sthe negative pressure in the second suction tube 753b may be released such that the egg is released into a chute 744 for disposal (see Fig. 13).

In a next step 816, the first and second sets of images are analysed in the image analysis system for signs of normal cell division, i.e. cell division corresponding to normal development of the egg. Because the two sets of images are captured in two substantially orthogonal planes and because the egg is rotated between images captures, at least one of the captured images will contains a clear view of the blastodisc 202, if present. If normal cell division is detected in the images, the egg is classified as fertilized. If no cell division is detected in the images, the egg is classified as un-fertilized. This step may advantageously employ computer vision and algorithms built by machine learning systems utilising mathematical models based on sample or training images of eggs known to be fertilized. The steps 810 to 816 are repeated for all eggs in the sample, thus yielding a fertilization rate for the sample.

As in the previously discussed embodiments, the image analysis system may comprise a central processing unit (CPU), volatile and/or non-volatile memory and data input and output devices. In the disclosed embodiment, the apparatus comprises an output device 758 in the form of a computer screen (see Fig. 10) on which the result of the analysis may be presented. The apparatus 700 may also comprise an input device (not shown), e.g. in the form of a keyboard, which may be used to input commands or instructions to control the apparatus 700.

In said image capturing steps 810 and 820, the lighting systems 724a and 724b ensure that sufficient lighting is provided.

In the preceding description, various aspects of the apparatus according to the invention have been described with reference to the illustrative embodiment. For purposes of explanation, specific numbers, systems and configurations were set forth in order to provide a thorough understanding of the apparatus and its workings. However, this description is not intended to be construed in a limiting sense. Various modifications and variations of the illustrative embodiment, as well as other embodiments of the apparatus, which are apparent to person skilled in the art to which the disclosed subject-matter pertains, may lie within the scope of the present invention as defined by the following claims.

## Claims

1. A method of identifying fertilized fish eggs (200) in a sample of eggs (104) taken from a batch of incubating fish eggs using the fish egg analysis system (100, 400) according to any one of claims 11-17, the method being **characterised by** the steps of:
- within a period of time from initial fertilization of the batch and using the fixation system (102), treating the sample (104) with a fixative (106) bringing an outer membrane (206) of the eggs at least partly transparent and/or a blastodisc (202) of the eggs at least partly opaque;
- using the at least one imaging system (108, 108', 108", 408, 708), capturing at least one image of individual eggs of the sample depicting the blastodisc (202) of the egg;
- using the image analysis system (112, 112', 412), analysing the at least one image for detecting divided cells in the blastodisc (202) of the egg; and
- identifying each egg associated with an image showing divided cells in the blastodisc (202) as a fertilized egg.

2. The method according to claim 1, wherein said step of capturing at least one image of individual eggs (200) comprises capturing a plurality of images of each egg from a plurality of directions.

3. The method according to claim 2, wherein said step of capturing at least one image of individual eggs (200) comprises capturing a first set of images of each individual egg (200) when rotated in a first plane and capturing a second set of images of the individual egg (200) when rotated in a second plane.

4. The method according to claim 3, wherein the second plane is orthogonal to the first plane.

5. The method according to any one of claims 3 and 4, wherein capturing the first set of images comprises capturing the first set of images with a first camera (722a) having an optical axis (723a) located in said first plane, and wherein capturing the second set of images comprises capturing the second set of images with a second camera (722b) having an optical axis (723b) located in said second plane.

6. The method according to any one of claims 3-5, wherein capturing any one of the first set of images and the second set of images comprises capturing images of said individual egg when it is rotated an entire revolution.

7. The method according to any one of claims 3-6, wherein capturing any one of the first set of images and the second set of images comprises rotating said individual egg between image captures within the range of 45° to 135°.

8. The method according to any one of the preceding claims, wherein said step of capturing at least one image of individual eggs (200) comprises bringing the eggs, one-by-one, passed said at least one imaging system (108, 108', 108").

9. The method according to any one of the preceding claims, wherein said period of time from initial fertilization of the batch to treating the sample (104) with the fixative (106) is within the range of 30 to 220 degree-hours, wherein said period of time is obtained by dividing the degree-hours value by the temperature, in centigrades, of the incubator of said batch of incubating fish eggs.

10. The method according to any one of the preceding claims, wherein said fixative comprises an acetic acid-alcohol-formaldehyde solution.

11. A fish egg analysis system (100, 400) comprising:
- at least one imaging system (108, 108', 108", 408, 708) being configured for capturing at least one image of individual fish eggs of a sample (104) of eggs comprising fertilized (200) and unfertilized fish eggs;
- an image analysis system (112, 112', 412); and
- a conveyor system (114, 414, 714) arranged for conveying the eggs of the sample past the at least one imaging system (108, 108', 108", 408, 708) in single file,
the fish egg analysis system (100, 400) being **characterised by:**
- a fixation system (102) configured for mixing the sample (104) with a fixative (106) bringing an outer membrane (206) of the eggs at least partly transparent and/or a blastodisc (202) of the eggs at least partly opaque;
- the at least one imaging system (108, 108', 108", 408, 708) being configured for depicting the blastodisc (202) of the eggs; and
- the image analysis system (112, 112', 412) being configured for analysing the captured images for detecting divided cells in the blastodisc (202) and identifying each egg associated with an image showing divided cells in the blastodisc (202) as a fertilized egg.

12. The system according to claim 11, **characterised in that** said at least one imaging system (708) comprises; a first camera (722a) being configured for capturing a first set of images of each of said individual fish eggs (200) when rotated in a first plane; and a second camera (722b) being configured for capturing a second set of images of the individual fish egg (200) when rotated in a second plane.

13. The system according to claim 12, **characterised in that** the second plane is orthogonal to the first plane.

14. The system according to any one of claims 12 and 13, **characterised in that** the first camera (722a) has an optical axis (723a) located in said first plane and the second camera (722b) has an optical axis (723b) located in said second plane.

15. The system (100) according to claim 11, **characterised in that** said at least one imaging system (108, 108', 108") comprises a first imaging system (108') and a second imaging system (108') being positioned one after the other along the conveyor system (114).

16. The system (100) according to any one of claims 11 and 15, **characterised in that** said at least one imaging system (108, 108', 108") is configured for capturing images of individual eggs (200) from a plurality of directions.

17. The system (100, 400) according to any one of claims 11-16, **characterised in that** said image analysis system (112, 112') employs an algorithm built by a machine learning system utilising mathematical models based on sample or training images of eggs known to be fertilized.

## Patentansprüche

1. Verfahren zum Identifizieren befruchteter Fischeier (200) in einer Probe von Eiern (104), die aus einer Charge bebrüteter Fischeier genommen wurde, unter Verwendung des Fischeieranalysesystems (100, 400) nach einem der Ansprüche 11-17, wobei das Verfahren durch die folgenden Schritte **gekennzeichnet ist:**
- innerhalb eines Zeitraums ab der anfänglichen Befruchtung der Charge und unter Verwendung des Fixierungssystems (102) Behandeln der Probe (104) mit einem Fixiermittel (106), das eine äußere Membran (206) der Eier mindestens teilweise transparent und/oder eine Keimscheibe (202) der Eier mindestens teilweise undurchsichtig macht;
- unter Verwendung des mindestens einen Bildgebungssystems (108, 108', 108", 408, 708) Erfassen mindestens eines Bildes einzelner Eier der Probe, die die Keimscheibe (202) des Eies darstellt;
- unter Verwendung des Bildanalysesystems (112, 112', 412) Analysieren des mindestens einen Bildes zum Erkennen geteilter Zellen in der Keimscheibe (202) des Eies; und
- Identifizieren jedes Eies, das einem Bild zugeordnet ist, das geteilte Zellen in der Keimscheibe (202) zeigt, als ein befruchtetes Ei.

2. Verfahren nach Anspruch 1, wobei der Schritt des Erfassens mindestens eines Bildes einzelner Eier (200) Erfassen einer Vielzahl von Bildern jedes Eies aus einer Vielzahl von Richtungen umfasst.

3. Verfahren nach Anspruch 2, wobei der Schritt des Erfassens mindestens eines Bildes einzelner Eier (200) Erfassen eines ersten Satzes von Bildern jedes einzelnen Eies (200), wenn es in einer ersten Ebene gedreht wird, und Erfassen eines zweiten Satzes von Bildern des einzelnen Eies (200), wenn es in einer zweiten Ebene gedreht wird, umfasst.

4. Verfahren nach Anspruch 3, wobei die zweite Ebene orthogonal zu der ersten Ebene ist.

5. Verfahren nach einem der Ansprüche 3 und 4, wobei das Erfassen des ersten Satzes von Bildern Erfassen des ersten Satzes von Bildern mit einer ersten Kamera (722a) mit einer optischen Achse (723a), die in der ersten Ebene liegt, umfasst, und wobei das Erfassen des zweiten Satzes von Bildern Erfassen des zweiten Satzes von Bildern mit einer zweiten Kamera (722b) mit einer optischen Achse (723b), die in der zweiten Ebene liegt, umfasst.

6. Verfahren nach einem der Ansprüche 3-5, wobei das Erfassen eines beliebigen von dem ersten Satz von Bildern und dem zweiten Satz von Bildern Erfassen von Bildern des einzelnen Eies umfasst, wenn es eine gesamte Umdrehung gedreht wird.

7. Verfahren nach einem der Ansprüche 3-6, wobei das Erfassen eines beliebigen von dem ersten Satz von Bildern und dem zweiten Satz von Bildern Drehen des einzelnen Eies zwischen Bilderfassungen innerhalb des Bereichs von 45° bis 135° umfasst.

8. Verfahren nach einem der vorhergehenden Ansprüche, wobei der Schritt des Erfassens mindestens eines Bildes einzelner Eier (200) Vorbeiführen der Eier nacheinander an dem mindestens einen Bildgebungssystem (108, 108', 108") umfasst.

9. Verfahren nach einem der vorhergehenden Ansprüche, wobei der Zeitraum ab der anfänglichen Befruchtung der Charge bis zum Behandeln der Probe (104) mit dem Fixiermittel (106) im Bereich von 30 bis 220 Grad-Stunden liegt, wobei der Zeitraum durch Teilen des Grad-Stunden-Werts durch die Temperatur in Celsius des Inkubators der Charge bebrüteter Fischeier erlangt wird.

10. Verfahren nach einem der vorhergehenden Ansprüche, wobei das Fixiermittel eine Essigsäure-Alkohol-Formaldehyd-Lösung umfasst.

11. Fischeieranalysesystem (100, 400) umfassend:
- mindestens ein Bildgebungssystem (108, 108', 108", 408, 708), das zum Erfassen mindestens eines Bildes einzelner Fischeier einer Probe (104) von Eiern, die befruchtete (200) und unbefruchtete Fischeier umfasst, konfiguriert ist;
- ein Bildanalysesystem (112, 112', 412); und
- ein Fördersystem (114, 414, 714), das angeordnet ist, um die Eier der Probe einzeln hintereinander an dem mindestens einen Bildgebungssystem (108, 108', 108", 408, 708) vorbeizubefördern,
wobei das Fischeieranalysesystem (100, 400) **gekennzeichnet ist:**
- durch ein Fixierungssystem (102), das zum Mischen der Probe (104) mit einem Fixiermittel (106) konfiguriert ist, das eine äußere Membran (206) der Eier mindestens teilweise transparent und/oder eine Keimscheibe (202) der Eier mindestens teilweise undurchsichtig macht;
- dadurch, dass das mindestens eine Bildgebungssystem (108, 108', 108", 408, 708) zum Darstellen der Keimscheibe (202) der Eier konfiguriert ist; und
- das Bildanalysesystem (112, 112', 412) zum Analysieren der erfassten Bilder zum Erkennen geteilter Zellen in der Keimscheibe (202) und zum Identifizieren jedes Eies, das einem Bild zugeordnet ist, das geteilte Zellen in der Keimscheibe (202) zeigt, als ein befruchtetes Ei konfiguriert ist.

12. System nach Anspruch 11, **dadurch gekennzeichnet, dass** das mindestens eine Bildgebungssystem (708) Folgendes umfasst; eine erste Kamera (722a), die dazu konfiguriert ist, einen ersten Satz von Bildern jedes der einzelnen Fischeier (200) zu erfassen, wenn es in einer ersten Ebene gedreht wird; und eine zweite Kamera (722b), die dazu konfiguriert ist, einen zweiten Satz von Bildern des einzelnen Fischeies (200) zu erfassen, wenn es in einer zweiten Ebene gedreht wird.

13. System nach Anspruch 12, **dadurch gekennzeichnet, dass** die zweite Ebene orthogonal zu der ersten Ebene ist.

14. System nach einem der Ansprüche 12 und 13, **dadurch gekennzeichnet, dass** die erste Kamera (722a) eine optische Achse (723a) aufweist, die in der ersten Ebene liegt, und die zweite Kamera (722b) eine optische Achse (723b) aufweist, die in der zweiten Ebene liegt.

15. System (100) nach Anspruch 11, **dadurch gekennzeichnet, dass** das mindestens eine Bildgebungssystem (108, 108', 108") ein erstes Bildgebungssystem (108') und ein zweites Bildgebungssystem (108') umfasst, die hintereinander entlang des Fördersystems (114) positioniert sind.

16. System (100) nach einem der Ansprüche 11 und 15, **dadurch gekennzeichnet, dass** das mindestens eine Bildgebungssystem (108, 108', 108") zum Erfassen von Bildern einzelner Eier (200) aus einer Vielzahl von Richtungen konfiguriert ist.

17. System (100, 400) nach einem der Ansprüche 11-16, **dadurch gekennzeichnet, dass** das Bildanalysesystem (112, 112') einen Algorithmus einsetzt, der durch ein maschinelles Lernsystem erstellt wurde, das mathematische Modelle verwendet, die auf Proben- oder Trainingsbildern von Eiern basieren, von denen bekannt ist, dass sie befruchtet sind.

## Revendications

1. Procédé d'identification d'œufs de poisson fécondés (200) dans un échantillon d'œufs (104) prélevé dans un lot d'œufs de poisson en incubation à l'aide du système d'analyse d'œufs de poisson (100, 400) selon l'une quelconque des revendications 11 à 17, le procédé étant **caractérisé par** les étapes de :
- dans un délai à partir de la fécondation initiale du lot et à l'aide du système de fixation (102), traitement de l'échantillon (104) avec un fixateur (106) rendant une membrane externe (206) des œufs au moins partiellement transparente et/ou un blastoderme (202) des œufs au moins partiellement opaque ;
- à l'aide de l'au moins un système d'imagerie (108, 108', 108", 408, 708), capture d'au moins une image d'œufs individuels de l'échantillon représentant le blastoderme (202) de l'œuf ;
- à l'aide du système d'analyse d'image (112, 112', 412), analyse de l'au moins une image pour détecter des cellules divisées dans le blastoderme (202) de l'œuf ; et
- identification de chaque œuf associé à une image montrant des cellules divisées dans le blastoderme (202) en tant qu'œuf fécondé.

2. Procédé selon la revendication 1, dans lequel ladite étape de capture d'au moins une image d'œufs individuels (200) comprend la capture d'une pluralité d'images de chaque œuf à partir d'une pluralité de directions.

3. Procédé selon la revendication 2, dans lequel ladite étape de capture d'au moins une image d'œufs individuels (200) comprend la capture d'un premier ensemble d'images de chaque œuf individuel (200) lorsqu'il est tourné dans un premier plan et la capture d'un second ensemble d'images de l'œuf individuel (200) lorsqu'il est tourné dans un second plan.

4. Procédé selon la revendication 3, dans lequel le second plan est orthogonal au premier plan.

5. Procédé selon l'une quelconque des revendications 3 et 4, dans lequel la capture du premier ensemble d'images comprend la capture du premier ensemble d'images avec une première caméra (722a) présentant un axe optique (723a) situé dans ledit premier plan, et dans lequel la capture du second ensemble d'images comprend la capture du second ensemble d'images avec une seconde caméra (722b) présentant un axe optique (723b) situé dans ledit second plan.

6. Procédé selon l'une quelconque des revendications 3 à 5, dans lequel la capture de l'un quelconque du premier ensemble d'images et du second ensemble d'images comprend la capture d'images dudit œuf individuel lorsqu'il est tourné d'un tour complet.

7. Procédé selon l'une quelconque des revendications 3 à 6, dans lequel la capture de l'un quelconque du premier ensemble d'images et du second ensemble d'images comprend la rotation dudit œuf individuel entre les captures d'images dans la plage de 45° à 135°.

8. Procédé selon l'une quelconque des revendications précédentes, dans lequel ladite étape de capture d'au moins une image d'œufs individuels (200) comprend le fait de faire passer les œufs, un par un, devant ledit au moins un système d'imagerie (108, 108', 108").

9. Procédé selon l'une quelconque des revendications précédentes, dans lequel ledit délai entre la fécondation initiale du lot et le traitement de l'échantillon (104) avec le fixateur (106) est dans la plage de 30 à 220 degrés-heures, dans lequel ledit délai est obtenu en divisant la valeur en degrés-heures par la température en centigrades de l'incubateur dudit lot d'œufs de poisson en incubation.

10. Procédé selon l'une quelconque des revendications précédentes, dans lequel ledit fixateur comprend une solution d'acide acétique-alcool-formaldéhyde.

11. Système d'analyse d'œuf de poisson (100, 400) comprenant :
- au moins un système d'imagerie (108, 108', 108", 408, 708) étant conçu pour capturer au moins une image d'œufs de poissons individuels d'un échantillon (104) d'œufs comprenant des œufs de poissons fécondés (200) et non fécondés ;
- un système d'analyse d'image (112, 112', 412) ; et
- un système de transport (114, 414, 714) agencé pour transporter les œufs de l'échantillon devant l'au moins un système d'imagerie (108, 108', 108", 408, 708) en un seule file,
le système d'analyse d'œuf de poisson (100, 400) étant **caractérisé par** :
- un système de fixation (102) conçu pour mélanger l'échantillon (104) avec un fixateur (106) rendant une membrane externe (206) des œufs au moins partiellement transparente et/ou un blastoderme (202) des œufs au moins partiellement opaque ;
- l'au moins un système d'imagerie (108, 108', 108", 408, 708) étant conçu pour représenter le blastoderme (202) des œufs ; et
- le système d'analyse d'image (112, 112', 412) étant conçu pour analyser les images capturées pour détecter des cellules divisées dans le blastoderme (202) et identifier chaque œuf associé à une image montrant des cellules divisées dans le blastoderme (202) en tant qu'œuf fécondé.

12. Système selon la revendication 11, **caractérisé en ce que** ledit au moins un système d'imagerie (708) comprend ; une première caméra (722a) étant conçue pour capturer un premier ensemble d'images de chacun desdits œufs de poisson individuels (200) lorsqu'il est tourné dans un premier plan ; et une seconde caméra (722b) étant conçue pour capturer un second ensemble d'images de l'œuf de poisson individuel (200) lorsqu'il est tourné dans un second plan.

13. Système selon la revendication 12, **caractérisé en ce que** le second plan est orthogonal au premier plan.

14. Système selon l'une quelconque des revendications 12 et 13, **caractérisé en ce que** la première caméra (722a) présente un axe optique (723a) situé dans ledit premier plan et la seconde caméra (722b) présente un axe optique (723b) situé dans ledit second plan.

15. Système (100) selon la revendication 11, **caractérisé en ce que** ledit au moins un système d'imagerie (108, 108', 108") comprend un premier système d'imagerie (108') et un second système d'imagerie (108') positionnés l'un après l'autre le long du système de transport (114).

16. Système (100) selon l'une quelconque des revendications 11 et 15, **caractérisé en ce que** ledit au moins un système d'imagerie (108, 108', 108") est conçu pour capturer des images d'œufs individuels (200) à partir d'une pluralité de directions.

17. Système (100, 400) selon l'une quelconque des revendications 11 à 16, **caractérisé en ce que** ledit système d'analyse d'image (112, 112') emploie un algorithme construit par un système d'apprentissage automatique utilisant des modèles mathématiques basés sur des images d'échantillon ou d'entraînement d'œufs connus comme étant fécondés.
